# EUROPEAN PATENT APPLICATION

(11) **EP 4 692 325 A1**
(43) Date of publication of application: **11.02.2026**
(21) Application number: 24193287.0
(22) Date of filing: 07.08.2024
(51) Int. Cl.: C12N 5/0783, G01N 33/50

(54) **METHOD FOR GENERATION OF A COMPOSITION COMPRISING NATURAL KILLER CELLS**

(71) Applicant: Miltenyi Biotec B.V. & Co. KG, 51429 Bergisch Gladbach (DE)
(72) Inventor: MEKES, Angela, 51429 Bergisch Gladbach (DE); SARISOY, Cem, 51429 Bergisch Gladbach (DE); ZHANG, Congcong, 51429 Bergisch Gladbach (DE)
(74) Representative: Biervert, Christian

(57) **Abstract**

The present invention discloses a method for generating a composition comprising NK cells using an obtained sample comprising leukocytes and platelets from a subject, wherein the leukocytes comprise at least NK cells and T cells, the method comprising a) depleting platelets from said sample, and b) depleting T cells from the sample of a).

## Description

### Field of the invention

The present invention generally relates to the field of separating immune cells, in particular to enriching Natural Killer (NK) cells from a sample comprising T cells and platelets such a whole blood sample.

### Background of the invention

Natural killer (NK) cells belong to the family innate lymphoid cells (ILCs). They are large granular lymphocytes (LGL) and are an important first line of defense against malignancies, viral infections and a broad range of pathogens. The activation of NK cells depends on their germ-line encoded activating and inhibitory receptors, therefore they can rapidly recognize and eliminate transformed cells without prior sensitization. NK cell infusion, either in combination with hematopoietic stem cell transplantation (HSCT) or as a stand-alone treatment, has been widely applied in the treatment of patients with cancer. In addition to autologous NK cells, the safety and efficacy of infusing allogeneic donor-derived NK cells into patients with leukemia has been demonstrated in the clinic. Donor-derived NK cells exhibit potent graft-versus tumor effects, but have low risk in inducing graft-versus-host-disease (GvHD).

In the allogeneic setting, however, alloreactive T cells cause GVHD, which occurs when T cells in the grafts recognize and attack the tissue cells as foreign in an immunosuppressed host. GvHD is a systemic disorder and can be fatal in the most severe cases. In allogeneic adoptive cell transfer (ACT) therapy, the most commonly used safety Standard is 5 × 10⁴ CD3+ T cells per kilogram (kg) of the patient's body weight. To ensure an engraftment without GVHD, a lower CD3+ T cell dose of 3 × 10⁴ cells per kg of the patient's body weight has been reported for fully haplotype mismatched transplants (Aversa et al., 1998, N Engl J Med. 1998 Oct 22;339(17):1186-93). It thereby indicates the importance of high degree of T cell depletion during the manufacturing of NK cell products.

There is a need in the art for an improved or alternative generation of a composition comprising NK cells that subsequently may be used in allogeneic adoptive cell transfer (ACT) therapy.

### Brief description of the invention

Because allogeneic T cells can induce GVHD in recipients, which can limit the use of NK cell products and cause potential dangers to patients, it is the aim to ensure a high degree of T cell depletion in NK cell products. Surprisingly, the inventors found the platelet to T cell ratio in the starting blood materials is negatively correlated with CD3+ T cell log-depletion. They developed processes to
efficiently remove platelets from starting blood materials, such as leukapheresis,
buffy coat, whole blood, cord blood and peripheral blood mononuclear cells (PBMCs).

High log-depletion of CD3+ T cells were achieved when sufficient platelets were removed from the starting blood materials.

Therefore, the present invention provides a method for generation of a composition comprising (enriched) NK cells from a sample comprising leukocytes and platelets from a subject, wherein the leukocytes comprise at least NK cells and T cells. The composition obtained by the method as disclosed herein is also provided.

### Brief description of the drawings

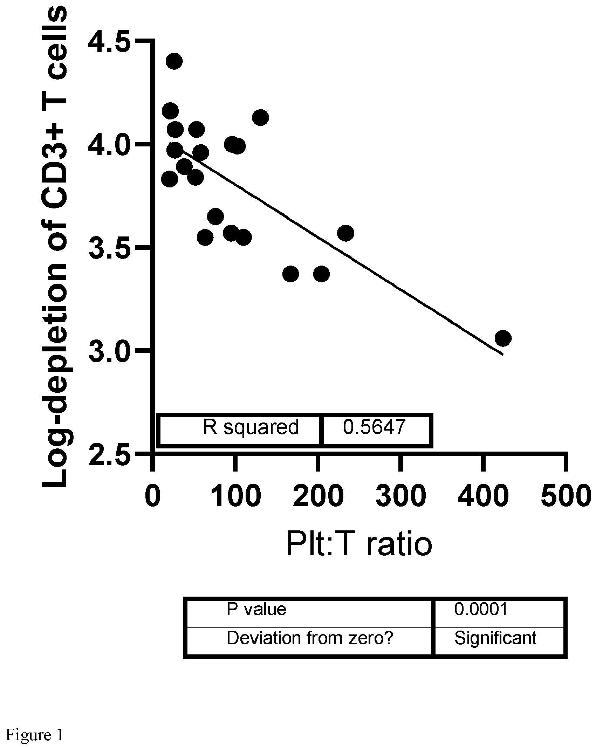

Figure 1: The CD3+ cell log depletion is negatively correlated with the ratio of platelets to T cells (Plt:T ratio) in the sample. Each symbol represents a distinct experiment. Data were analyzed by linear regression (y=-0,002541x + 4,058, *p*=0.0001 for deviation from zero); the coefficient of determination R squared shows the correlation between Log-depletion of CD3+ T cells and Plt:T ratio.

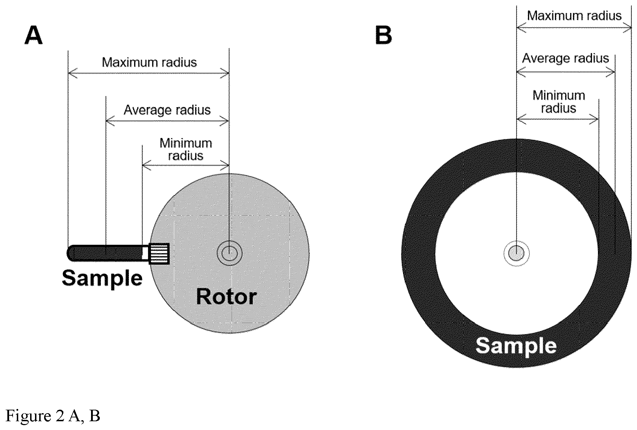

Figure 2: Schematic representations of the maximum, average and minimum radii determined by the distance between the axis of rotation of a centrifuge and the top, middle, and bottom of a sample, respectively. The radius measurement is shown for a centrifuge with a rotor (A) or a centrifugation chamber (B).

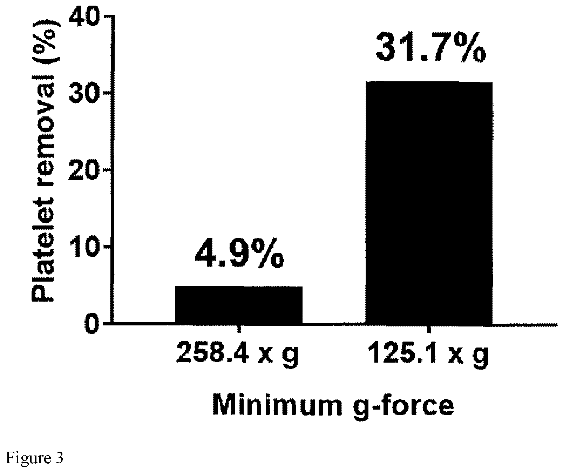

Figure 3: The importance of minimum g-force for efficient platelet removal in blood samples by centrifugation washing. The blood sample that was centrifuged with the minimum g-force of 125.1 x g showed more efficient platelet removal, compared with the blood sample that was centrifuged with the minimum g-force of 258.4 x g, although the same maximum g-force of 322 x g was applied to both samples in the centrifugation chamber.

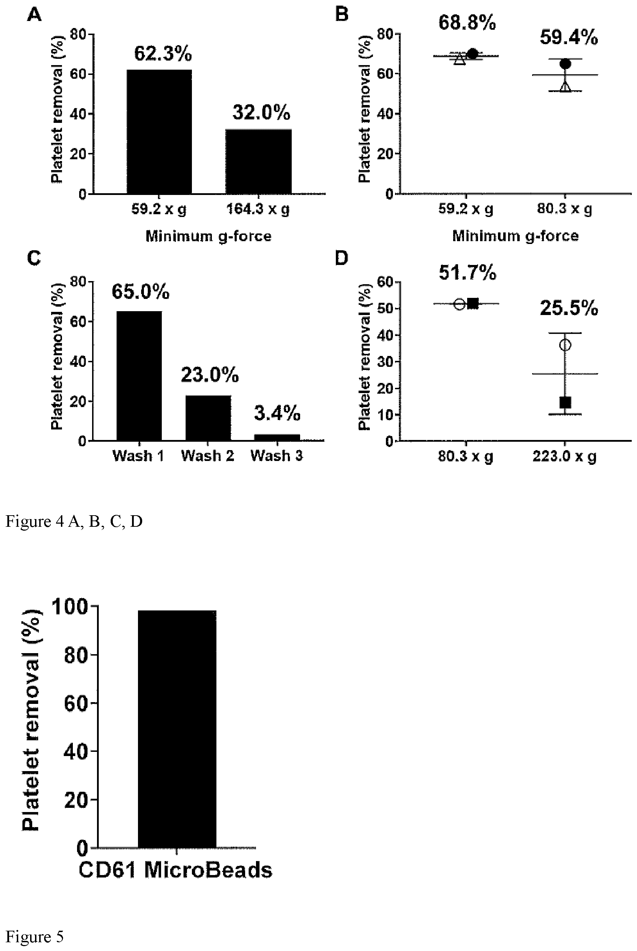

Figure 4: Optimization of the centrifugation washing method to improve the platelet removal efficiency. (A) Comparison between the minimum g-force of 59,2 x g and 164.3 x g for a buffy coat sample. (B) Comparison between the minimum g-force of 59,2 x g and 80.3 x g for the buffy coat samples from two donors. Mean values ± SD are shown. (C) Three washing rounds were applied to one buffer coat sample centrifuged with the minimum g-force of 80.3 x g shown in (B). The platelet removal efficiency was calculated after each wash based on the amount of platelets in the starting blood material. (D) Comparison between the minimum g-force of 80,3 x g and 223.0 x g for the leukaphereses from two donors. Mean values ± SD are shown.
Figure 5: Efficient platelet removal can be achieved by magnetic cell separation technology.

### Detailed description of the invention

In a first aspect the present invention provides a (in-vitro) method for generating a composition (for subsequent processing) comprising (enriched) NK cells using an obtained sample comprising leukocytes and platelets from a subject, wherein the leukocytes comprise at least NK cells and T cells, the method comprising
a) Depleting platelets from said sample, and
b) Depleting T cells from the sample of a),
(thereby enriching NK cells in said composition).

The obtained sample may be e.g. leukapheresis, buffy coat, whole blood, cord blood or peripheral blood mononuclear cells (PBMCs).

Said method, wherein the ratio of platelets to T cells in the sample that is depleted of platelets (the sample of step a)) is less than 150, less than 100, less than 50 or less than 30.

Said method, wherein said depletion of said T cells from the sample of a) is the depletion of T cells that express CD3.

Said method, wherein said depleting of said platelets from said sample is performed by
i) Washing by centrifugation, or
ii) Affinity chromatography using CD61 as a selection marker

CD61 is expressed on the surface of platelets, therefore CD61 is a platelet selection marker.

Said method, wherein said washing by centrifugation is performed in such a way that in said sample comprising leukocytes and platelets platelets are separated from lymphocytes.

Said method, wherein said washing by centrifugation is performed in such a way that in said sample comprising leukocytes and platelets the speed of rotation is lower than that used to pellet the platelets but pellets the lymphocytes.

The skilled person knows how to enrich platelets from a sample such as PBMC by centrifugation, see e.g. Perez AG et al. (Hematol. 2014 Mar 25;2014:176060) . Therefore, the skilled person also knows how to separate platelets from lymphocytes from a sample such as PBMC and subsequently harvest the lymphocytes without the platelets.

Said method, wherein said washing by centrifugation is performed by using a minimum g force between 25 x g and 200 x g

The g-force or gravitational force equivalent is mass-specific force (force per unit mass), expressed in units of standard gravity (symbol g).

The minimum g-force is calculated from the minimum radius, which is determined by the distance between the axis of rotation of a centrifuge and the top of a sample.

Said method, wherein said washing by centrifugation is performed at least once.

Said method, wherein said washing by centrifugation is performed at least twice.

Said method, wherein the method comprises
a) Depleting platelets from said sample, and
b) Depleting T cells from the sample of a) by depletion of cells that are CD3+.

Said method, wherein the depletion of T cells is performed by using an antigen binding domain specific for the antigen CD3 or specific for the alpha/betaTCR.

Said antigen binding domain may be an antibody or antigen binding fragment thereof.

Said method, wherein said depletion of T cells is performed by magnetic cell separation , and wherein said antigen binding domain specific for the antigen CD3 or for the alpha/beta TCR is directly or indirectly coupled to magnetic beads.

Said method, wherein the method comprises
a) Depleting platelets from said sample, and
b) Depleting T cells from the sample of a) by depletion cells that are CD3+, and wherein the method additionally comprises:
   c1) further depleting cells that are CD19+ and/or CD14+,
wherein the step c1) may be performed simultaneously with step b) or after step b), or c2) enriching cells after step b) that are CD56+.

Said method, wherein the depletion of CD19+ cells is performed by using an antigen binding domain specific for the antigen CD19.

Said antigen binding domain may be an antibody or antigen binding fragment thereof.

Said method, wherein said depletion of CD19+ cells is performed by magnetic cell separation, and wherein said antigen binding domain specific for the antigen CD19 is directly or indirectly coupled to magnetic beads.

Said method, wherein the depletion of CD14+ cells is performed by using an antigen binding domain specific for the antigen CD14.

Said antigen binding domain may be an antibody or antigen binding fragment thereof.

Said method, wherein said depletion of CD14+ cells is performed by magnetic cell separation, and wherein said antigen binding domain specific for the antigen CD14 is directly or indirectly coupled to magnetic beads.

Said method, wherein the enrichment of CD56+ cells is performed by using an antigen binding domain specific for the antigen CD56.

Said antigen binding domain may be an antibody or antigen binding fragment thereof.

Said method, wherein said depletion of CD56+ cells is performed by magnetic cell separation, and wherein said antigen binding domain specific for the antigen CD56 is directly or indirectly coupled to magnetic beads.

Said method, wherein said affinity chromatography is performed by using an antigen binding domain specific for the antigen CD61 (thereby depleting the platelets from said sample comprising leukocytes and platelets).

Said method, wherein said affinity chromatography is performed by magnetic cell separation, and wherein said antigen binding domain specific for the antigen CD61 is directly or indirectly coupled to magnetic beads.

Said method as disclosed herein, wherein the method is performed in a closed system.

Said method, wherein the method is performed in a closed system, and wherein said method is an automated method.

Said method, wherein the method comprises the additional step:
genetically modifying of the enriched NK cells.

Said genetically modifying may be after step b) (after depleting T cells from the sample of a) by depletion cells that are CD3+) (when there is no step c1 or c2).

Said genetically modifying may be after step c) (after further depleting cells that are CD19+ and/or CD14+, or after enriching cells after step b) that are CD56+) (when there is a step c1 or c2).

Said method, wherein said method additionally comprises a step of expansion/proliferation and/or cultivation of the NK cells before and/or after the genetic modification step.

Said method, where said method additionally comprises a step of cryopreservation of the NK cells.

Cryopreservation or cryoconservation is a process that preserves biological materials, such as organelles, cells, tissues, or any other biological samples, by subjecting the samples to very low temperatures, such as -80 °C (-112 °F) or -196 °C (-321 °F) using liquid nitrogen.

The terms "genetically modified immune cell (NK cell)" or "engineered immune cell (NK cell)" may be used interchangeably and mean containing and/or expressing a foreign gene or nucleic acid sequence which in turn modifies the genotype or phenotype of the cell or its progeny. These terms also may include the gene-knockout, gene knock-in or gene-corrected NK cells generated by endonucleases such as CRISPR/Cas technology. Especially, the terms refer to the fact that cells can be manipulated by recombinant methods well known in the art to express stably or transiently peptides or proteins, e.g. CARs which are not expressed in these cells in the natural state. Genetic modification of cells may include but is not restricted to transfection, electroporation, nucleofection, transduction using retroviral vectors, lentiviral vectors, non-integrating retro- or lentiviral vectors, transposons, designer nucleases including zinc finger nucleases, TALENs or CRISPR/Cas.

Said genetically modifying of the NK cells may be the transduction, the electroporation, or transfection of the NK cells.

Said genetically modifying of the NK cells may be the transduction of the NK cells with a retroviral vector such as a lentiviral vector.

The transfection may be the transfection of NK cells with lipid nanoparticles.

The transfection of the NK cells may be the introduction of specific DNA sequences by the Sleeping Beauty transposon system into the NK cells.

The engineered NK cell may be modified by genetic engineering using methods well known in the art e.g. Meganucleases, TALEN, CrisprCas, zinc finger nucleases, shRNA and /or miRNA. Said engineered NK cells may be modified e.g. to reduce or lack expression of a specific gene, which is normally expressed in the NK cell.

Said engineered NK cells may express a chimeric antigen receptor (CAR) or a T cell receptor (TCR)

Said NK cells expressing e.g. a CAR or TCR may be modified to express additional transgenes such as therapeutic controls, cytokines and/or fragments, cytokine receptors and/or fragments, cytokine receptor fusion proteins, costimulatory receptors, transcription factors or armoring molecules such as, but not limited to, metalloproteases/ECM-degrading enzymes.

In another aspect the present invention provides a composition comprising NK cells, wherein said composition is obtained by the methods as disclosed herein.

In another aspect the present invention provides a composition for use in treatment of a disease such as cancer, an autoimmune disease or an infectious disease, the composition comprising NK cells, wherein said composition is obtained by the methods as disclosed herein.

In another aspect the present invention provides a pharmaceutical composition comprising NK cells, wherein said composition is obtained by the methods as disclosed herein, and optionally a pharmaceutically acceptable carrier.

Pharmaceutically acceptable carriers, diluents or excipients may comprise buffers such as neutral buffered saline, phosphate buffered saline and the like; carbohydrates such as glucose, mannose, sucrose or dextrans, mannitol; proteins; polypeptides or amino acids such as glycine; antioxidants; chelating agents such as EDTA or glutathione; adjuvants (e.g., aluminum hydroxide); and preservatives.

In one embodiment of the invention the composition comprising NK cells, wherein said composition is obtained by the methods as disclosed herein immune cells and wherein said NK cells have been modified to express a CAR as disclosed herein is for use in treatment of a disease associated with a target cell of a subject suffering from said disease such as a cancer, wherein said target cell may be a leukemia or lymphoma or solid cancer (said composition is referred to here as the "disclosed composition"). NK cells of a subject may be enriched as disclosed herein. The subject may e.g. suffer from said cancer or may be a healthy subject. These NK cells may be genetically modified *in vitro* to express the CAR as disclosed herein. These engineered NK cells of said disclosed composition may be activated and expanded *in vitro.* In a cellular therapy said disclosed composition may be infused to a recipient in need thereof. Said composition may be a pharmaceutical composition (said cell plus pharmaceutical acceptable carrier). The infused NK cells of said composition may be e.g. able to kill (or at least stop growth of) cancerous cells in the recipient. The recipient may be the same subject from which the cells was obtained (autologous cell therapy) or may be from another subject of the same species (allogeneic cell therapy).

Said composition comprising the NK cells engineered to express a CAR as disclosed herein may be administered either alone, or as a pharmaceutical composition in combination with diluents and/or with other components such as IL-2 or other cytokines or cell populations. Briefly, pharmaceutical compositions of said disclosed composition may be in combination with one or more pharmaceutically or physiologically acceptable carriers, diluents or excipients. Such compositions may comprise buffers such as neutral buffered saline, phosphate buffered saline and the like; carbohydrates such as glucose, mannose, sucrose or dextrans, mannitol; proteins; polypeptides or amino acids such as glycine; antioxidants; chelating agents such as EDTA or glutathione; adjuvants (e.g., aluminum hydroxide); and preservatives.

Preferentially, the disclosed compositions are formulated for intravenous administration. The administration of cell compositions to the subject may be carried out in any convenient manner known in the art.

Pharmaceutical compositions of the disclosed compositions may be administered in a manner appropriate to the disease to be treated. Appropriate dosages may be determined by clinical trials. But the quantity and frequency of administration will also be determined and influenced by such factors as the condition of the patient, and the type and severity of the patient's disease.

A pharmaceutical composition comprising the disclosed composition comprising the NK cells as disclosed herein may be administered at a dosage of 10⁴ to 10⁹ cells/kg body weight, preferably 10⁵ to 10⁷ cells/kg body weight. The disclosed compositions may also be administered several times at these dosages. The disclosed compositions of cells may be injected e.g. directly into a tumor, lymph node, or site of infection.

The disclosed composition may be used in combination with e.g. chemotherapy, radiation, immunosuppressive agents, antibodies or antibody therapies.

### Generation of NK cells

Processes of generation of a composition of NK cells are well known in the art. Exemplarily in the following methods of generation of immune cells expressing a CAR are disclosed.

Generally, other processes of enrichment of NK cells than the method disclosed herein are known in the art and such enrichments of NK cells may be performed by e.g. enrichment of CD56+ cells from a sample obtained from a subject such as a blood sample or PBMC, followed by depletion of CD3+ cells from said sample. Alternatively said enrichment of NK cells may be the CD3 depletion of cells from said sample. Alternatively said enrichment of NK cells may be the CD3 and CD19 and/or CD14 depletion of cells from said sample. Alternatively, said enrichment may be the depletion of CD3+ cells followed by the enrichment of CD56+ cells from said sample.

NK cells can be further cultivated and expanded by methods known in the art with cytokines such as IL-2, IL-15, IL-21, IL-12, IL-18, IL-1 family, and/or with feeder cells or artificial antigen presenting cells (APCs) such as K562-based feeder cells, EBV transformed lymphoblastoid cell lines, PMBCs, and/or cell-free derivatives such as cell particles or exosomes, and/or with stimulating reagents such as NK cell activation/expansion kit from Miltenyi Biotec, ImmunoCult^{™} NK Cell Expansion Kit from StemCell Technologies, CellXVivo Human NK Cell Expansion Kit from R&D Systems. NK cells can be cultivated and expanded by methods known in the art with culturing flasks, culturing bags, G-Rex, bioreactors, wave cell culture system, etc.

### Modification of NK cells

### Nucleotides, Expression, Vectors

The nucleic acids encoding a transgene such as a CAR as used herein may e.g. comprise a nucleotide sequence encoding any of the leader sequences, antigen binding domains, transmembrane domains, and/or intracellular T cell signaling domains described herein for CARs.

In some embodiments, the nucleotide sequence may be codon-modified. Without being bound to a particular theory, it is believed that codon optimization of the nucleotide sequence increases the translation efficiency of the mRNA transcripts. Codon optimization of the nucleotide sequence may involve substituting a native codon for another codon that encodes the same amino acid, but can be translated by tRNA that is more readily available within a cell, thus increasing translation efficiency. Optimization of the nucleotide sequence may also reduce secondary mRNA structures that would interfere with translation, thus increasing translation efficiency.

"Nucleic acid" as used herein includes "polynucleotide", "oligonucleotide", "nucleic acid molecule" and "nucleic acid sequence" and generally means a polymer of DNA or RNA, which can be single-stranded or double-stranded, synthesized or obtained (e.g., isolated and/or purified) from natural sources, which can contain natural, non-natural or altered nucleotides, and which can contain a natural, non-natural or altered internucleotide linkage, such as a phosphoroamidate linkage or a phosphorothioate linkage, instead of the phosphodiester found between the nucleotides of an unmodified oligonucleotide.

In an embodiment, the nucleic acids can be incorporated into a recombinant expression vector. In this regard, an embodiment provides recombinant expression vectors comprising any of the nucleic acids.

For purposes herein, the term "recombinant expression vector" means a genetically-modified oligonucleotide or polynucleotide construct that permits the expression of an mRNA, protein, polypeptide, or peptide by a host cell, when the construct comprises a nucleotide sequence encoding the mRNA, protein, polypeptide, or peptide, and the vector is contacted with the cell under conditions sufficient to have the mRNA, protein, polypeptide, or peptide expressed within the cell. The vectors are not naturally-occurring as a whole.

In an embodiment, the recombinant expression vector can be any suitable recombinant expression vector, and can be used to transform or transfect any suitable host cell. Suitable vectors include those designed for propagation and expansion or for expression or both, such as plasmids and viruses.

The recombinant expression vector may be a viral vector, e.g., a retroviral vector or a lentiviral vector. A lentiviral vector is a vector derived from at least a portion of a lentivirus genome, including especially a self-inactivating lentiviral vector. Other examples of lentivirus vectors that may be used in the clinic, include, for example, and not by way of limitation, the LENTIVECTOR.RTM. gene delivery technology from Oxford BioMedica plc, the LENTIMAX.TM. vector system from Lentigen and the like. Nonclinical types of lentiviral vectors are also available and would be known to one skilled in the art.

A number of transfection techniques are generally known in the art. Transfection methods include e.g. calcium phosphate co-precipitation, direct micro injection into cultured cells, electroporation, liposome mediated gene transfer, and lipid mediated transduction. If DNA or RNA is introduced into cells by using viral vector carriers, then the technique is called transduction.

Constructs of expression vectors, which are circular or linear, can be prepared to contain a replication system functional in a prokaryotic or eukaryotic host cell.

The recombinant expression vector may comprise regulatory sequences, such as transcription and translation initiation and termination codons, which are specific to the type of host cell (e.g., bacterium, fungus, plant, or animal) into which the vector is to be introduced, as appropriate, and taking into consideration whether the vector is DNA- or RNA-based. The recombinant expression vector may comprise restriction sites to facilitate cloning.

The recombinant expression vector can include one or more marker genes, which allow for selection of transformed or transfected host cells. Marker genes include biocide resistance, e.g., resistance to antibiotics, heavy metals, etc., complementation in an auxotrophic host to provide prototrophy, and the like. Suitable marker genes for the inventive expression vectors include, for instance, neomycin/G418 resistance genes, hygromycin resistance genes, histidinol resistance genes, tetracycline resistance genes, and ampicillin resistance genes.

The recombinant expression vector can comprise a native or nonnative promoter operably linked to the nucleotide sequence encoding the CAR (including functional portions and functional variants thereof), or to the nucleotide sequence which is complementary to or which hybridizes to the nucleotide sequence encoding the CAR. The selection of promoters, e.g., strong, weak, inducible, tissue-specific and developmental-specific, is within the ordinary skill of the artisan. Similarly, the combining of a nucleotide sequence with a promoter is also within the skill of the artisan. The promoter can be a nonviral promoter or a viral promoter, e.g., a cytomegalovirus (CMV) promoter, an SV40 promoter, an RSV promoter, human elongation factor-1 alpha (EF-1 alpha) promoter or a promoter found in the long-terminal repeat of the murine stem cell virus.

The recombinant expression vectors can be designed for either transient expression, for stable expression, or for both. Also, the recombinant expression vectors can be made for constitutive expression or for inducible expression.

Further, the recombinant expression vectors can be made to include a suicide gene. As used herein, the term "suicide gene" refers to a gene that causes the cell expressing the suicide gene to die. The suicide gene can be a gene that confers sensitivity to an agent, e.g., a drug, upon the cell in which the gene is expressed, and causes the cell to die when the cell is contacted with or exposed to the agent. Suicide genes are known in the art and include, for example, the Herpes Simplex Virus (HSV) thymidine kinase (TK) gene, cytosine deaminase, purine nucleoside phosphorylase, and nitroreductase.

### Processes for modification of NK cells

The composition of NK cells obtained by the method as disclosed herein may be genetically modified to express one or more transgenes, and/or to knock out endogenous genes of the NK cells.

Processes of genetically modifying NK cells are well known in the art. Exemplarily in the following methods of generation of NK cells expressing a transgene such as a CAR are disclosed

A process for the generation of genetically modified NK cells with a pseudotyped retroviral vector particle thereof is disclosed e.g. in WO2019121945A1 and may comprise the e.g. steps:
a) Activation of NK cells, and
b) Addition of a pseudotyped retroviral vector particle or virus-like particle thereof to said activated NK cells,

wherein said pseudotyped retroviral vector particle comprises a modified baboon endogenous retrovirus (BaEV) envelope glycoprotein that is able of binding to and fusing with a hematopoietic cell membrane, thereby transferring biological material into said activated NK cells,
wherein said modified baboon endogenous retrovirus (BaEV) envelope glycoprotein that is able of binding to and fusing with a hematopoietic cell membrane is:
   a chimeric envelope glycoprotein which comprises or consists in a fusion of the transmembrane and extracellular domain of a baboon endogenous retrovirus (BaEV) envelope glycoprotein and the cytoplasmic tail domain of a murine leukemia virus (MLV) envelope glycoprotein; or
   a modified BaEV envelope glycoprotein wherein the cytoplasmic tail domain is devoid of the fusion inhibitory R peptide,
   and wherein said pseudotyped retroviral vector particle comprises a nucleic acid encoding a transgene such as a CAR.

Said activation of said NK cells may be achieved by the addition of at least one cytokine or feeder cells or membrane particles of feeder cells or a with an NK cell activation reagent to said NK cells.

Said at least one cytokine may be IL-2 and/or IL-15.

Said activation of NK cells may be achieved by the addition of a combination of cytokines comprising at least one cytokine that activates NK cells and a IL-1 family cytokine.

Said combination of cytokines may be IL2 and/or IL-15 and a IL-1 family cytokine.

Said IL-1 family cytokine may be IL-18, IL-33 or IL-1beta.

### General methods of treatment using the compositions as disclosed herein

It is contemplated that both the compositions of enriched NK cells as disclosed herein and the composition of enriched NK cells and subsequently genetically modified as disclosed herein can be used in methods of treating or preventing a disease in a mammal. In this regard, an embodiment provides a method of treating cancer administering to the mammal (the subject) the compositions of enriched NK cells as disclosed herein or the composition of enriched NK cells and subsequently genetically modified as disclosed herein, wherein the modified NK cells express a CAR or TCR specific for an TAA of said cancer. The compositions may comprise (modified) NK cells in an amount effective to treat cancer in the mammal.

An embodiment, especially for the treatment of cancer, further comprises lymphodepleting the mammal prior to administering the compositions disclosed herein. Examples of lymphodepletion include, but may not be limited to, nonmyeloablative lymphodepleting chemotherapy, myeloablative lymphodepleting chemotherapy, total body irradiation, etc.

For purposes of the methods, wherein NK cells are administered, the cells can be cells that are allogeneic or autologous to the mammal. Preferably, the cells are allogeneic to the mammal. As used herein, allogeneic means any material derived from a different animal of the same species as the individual to whom the material is introduced. Two or more individuals are said to be allogeneic to one another when the genes at one or more loci are not identical. In some aspects, allogeneic material from individuals of the same species may be sufficiently unlike genetically to interact antigenically. As used herein, "autologous" means any material derived from the same individual to whom it is later to be re-introduced into the individual.

The mammal referred to herein can be any mammal. As used herein, the term "mammal" refers to any mammal, including, but not limited to, mammals of the order Rodentia, such as mice and hamsters, and mammals of the order Logomorpha, such as rabbits. The mammals may be from the order Carnivora, including Felines (cats) and Canines (dogs). The mammals may be from the order Artiodactyla, including Bovines (cows) and Swines (pigs) or of the order Perssodactyla, including Equines (horses). The mammals may be of the order Primates, Ceboids, or Simoids (monkeys) or of the order Anthropoids (humans and apes). Preferably, the mammal is a human.

The terms "treat," and "prevent" as well as words stemming therefrom, as used herein, do not necessarily imply 100% or complete treatment or prevention. Rather, there are varying degrees of treatment or prevention of which one of ordinary skill in the art recognizes as having a potential benefit or therapeutic effect. In this respect, the methods can provide any amount or any level of treatment of cancer in a mammal.

Any method of administration can be used for the disclosed therapeutic agents, including local and systemic administration. For example topical, oral, intravascular such as intravenous, intramuscular, intraperitoneal, intranasal, intradermal, intrathecal and subcutaneous administration can be used. The particular mode of administration and the dosage regimen will be selected by the attending clinician, taking into account the particulars of the case (for example the subject, the disease, the disease state involved, and whether the treatment is prophylactic). In cases in which more than one agent or composition is being administered, one or more routes of administration may be used; for example, a chemotherapeutic agent may be administered orally and a composition of NK cells expressing a CARs as disclosed herein may be administered intravenously.

Methods of administration include injection for which the compositions as disclosed herein are provided in a nontoxic pharmaceutically acceptable carrier such as water, saline, Ringer's solution, dextrose solution, 5% human serum albumin, fixed oils, ethyl oleate, or liposomes. In some embodiments, local administration of the disclosed compounds or compositions (e.g. the NK cells expressing a CAR as disclosed herein) can be used, for instance by applying the compounds or compositions to a region of tissue from which a tumor has been
removed, or a region suspected of being prone to tumor development. In some embodiments, sustained intra-tumoral (or near-tumoral) release of the pharmaceutical preparation that includes a therapeutically effective amount of the compounds or compositions may be beneficial. In other examples, the conjugate is applied as an eye drop topically to the cornea, or intravitreally into the eye.

The disclosed therapeutic agents can be formulated in unit dosage form suitable for individual administration of precise dosages. In addition, the disclosed therapeutic agents may be administered in a single dose or in a multiple dose schedule. A multiple dose schedule is one in which a primary course of treatment may be with more than one separate dose, for instance 1-10 doses, followed by other doses given at subsequent time intervals as needed to maintain or reinforce the action of the compositions.

Treatment can involve daily or multi-daily doses of compound(s) over a period of a few days to months, or even years. Thus, the dosage regime will also, at least in part, be determined based on the particular needs of the subject to be treated and will be dependent upon the judgment of the administering practitioner.

All definitions, characteristics and embodiments defined herein with regard to the first aspect of the invention as disclosed herein also apply mutatis mutandis in the context of the other aspects of the invention as disclosed herein.

### Definitions

Unless defined otherwise, technical and scientific terms used herein have the same meaning as commonly understood by one of ordinary skill in the art to which this invention belongs.

As used herein the term "comprising" or "comprises" is used in reference to compositions, methods, and respective component(s) thereof, that are essential to the method or composition, yet open to the inclusion of unspecified elements, whether essential or not.

As used herein, the term "about" will be understood by persons of ordinary skill in the art and will vary to some extent on the context in which it is used. As used herein, "about" when referring to a measurable value such as an amount, a temporal duration, and the like, is meant to encompass variations of ±20% or ±10%, more preferably ±5%, even more preferably ±1% from the specified value

The term "antibody" as used herein is used in the broadest sense to cover the various forms of antibody structures including but not being limited to monoclonal and polyclonal antibodies (including full length antibodies), multispecific antibodies (e.g. bispecific antibodies), antibody fragments, i.e. antigen binding fragments of an antibody, immunoadhesins and antibody-immunoadhesin chimeras, that specifically recognize (i.e. bind) an antigen. "Antigen binding fragments" comprise a portion of a full-length antibody, preferably the variable domain thereof, or at least the antigen binding site thereof ("an antigen binding fragment of an antibody"). Examples of antigen binding fragments include Fab (fragment antigen binding), scFv (single chain fragment variable), single domain antibodies (nanobodies), diabodies, dsFv, Fab', F(ab')2, diabodies, single-chain antibody molecules, and multispecific antibodies formed from antibody fragments. The antibody or antibody fragment may be human, fully human, humanized, human engineered, non-human, and/or chimeric. The non-human antibody or antibody fragment may be humanized to reduce immunogenicity to humans, while retaining the specificity and affinity of the parental non-human antibody. Chimeric antibodies may refer to antibodies created through the joining of two or more antibody genes which originally encoded for separate antibodies.

The terms "having specificity for", "specifically binds" or "specific for" with respect to an antigen-binding domain of an antibody, of a fragment thereof or of a CAR refer to an antigen-binding domain which recognizes and binds to a specific antigen, but does not substantially recognize or bind other molecules in a sample. An antigen-binding domain that binds specifically to an antigen from one species may bind also to that antigen from another species. This cross-species reactivity is not contrary to the definition of that antigen-binding domain is specific. An antigen-binding domain that specifically binds to an antigen may bind also to different allelic forms of the antigen (allelic variants, splice variants, isoforms etc.). This cross reactivity is not contrary to the definition of that antigen-binding domain is specific.

As used herein, the term "antigen" is intended to include substances that bind to or evoke the production of one or more antibodies and may comprise, but is not limited to, proteins, peptides, polypeptides, oligopeptides, lipids, carbohydrates such as dextran, haptens and combinations thereof, for example a glycosylated protein or a glycolipid. The term "antigen" as used herein refers to a molecular entity that may be expressed e.g. on the surface of a target cell and that can be recognized by means of the adaptive immune system including but not restricted to antibodies or TCRs, or engineered molecules including but not restricted to endogenous or transgenic TCRs, CARs, scFvs or multimers thereof, Fab-fragments or multimers thereof, antibodies or multimers thereof, single chain antibodies or multimers thereof, or any other molecule that can execute binding to a structure with high affinity.

The terms "immune cell" or "immune effector cell" may be used interchangeably and refer to a cell that may be part of the immune system and executes a particular effector function such as alpha-beta T cells, NK cells, NKT cells, B cells, innate lymphoid cells (ILC), cytokine induced killer (CIK) cells, lymphokine activated killer (LAK) cells, gamma-delta T cells, regulatory T cells (Treg), monocytes or macrophages. Preferentially these immune cells are human immune cells. Preferred immune cells are cells with cytotoxic effector function such as alpha-beta T cells, NK cells, NKT cells, ILC, CIK cells, LAK cells or gamma-delta T cells. Most preferred immune effector cells are T cells and NK cells. Tumor infiltrating lymphocytes (TILs) are T cells that have moved from the blood of a subject into a tumor. These TILs may be removed from a patient's tumor by methods well known in the art, e.g. enzymatic and mechanic tumor disruption followed by density centrifugation and/or cell marker specific enrichment. TILs are genetically engineered as disclosed herein, and then given back to the patient. "Effector function" means a specialized function of a cell, e.g. in a T cell an effector function may be cytolytic activity or helper activity including the secretion of cytokines.

Immunotherapy is a medical term defined as the "treatment of disease by inducing, enhancing, or suppressing an immune response". Immunotherapies designed to elicit or amplify an immune response are classified as activation immunotherapies, while immunotherapies that reduce or suppress are classified as suppression immunotherapies. Cancer immunotherapy as an activating immunotherapy attempts to stimulate the immune system to reject and destroy tumors. Adoptive cell transfer uses cell-based, preferentially T cell-based or NK cell-based cytotoxic responses to attack cancer cells. For example, T cells or NK cells that have a natural or genetically engineered reactivity to a patient's cancer are generated in-vitro and then transferred into the cancer patient. Then the immunotherapy is referred to as "CAR cell immunotherapy" or in case of use of T cells only as "CAR T cell therapy" or "CAR T cell immunotherapy" or in case of use of NK cells only as "CAR NK cell therapy" or "CAR NK cell immunotherapy"..

The term "treatment" as used herein means to reduce the frequency or severity of at least one sign or symptom of a disease.

The term "autologous" as used herein refers to any material derived from the same subject to who it is later re-introduced.

The term "allogeneic" as used herein refers to any material derived from a different subject of the same species as the subject to who the material is re-introduced.

The terms "therapeutically effective amount" or "therapeutically effective population" mean an amount of a cell population which provides a therapeutic benefit in a subject.

As used herein, the term "subject" refers to an animal. Preferentially, the subject is a mammal such as mouse, rat, cow, pig, goat, chicken dog, monkey or human. More preferentially, the subject is a human. The subject may be a subject suffering from a disease such as cancer (a patient) or from an autoimmune disease or from an allergic disease or from an infectious disease or from graft rejection.

The term "expression" as used herein is defined as the transcription of a particular nucleotide sequence into RNA and optionally subsequent translation of said RNA into a polypeptide sequence or a protein.

A "vector" comprises a (isolated) nucleic acid molecule which can be used to deliver the (isolated) nucleic acid molecule to the interior of a cell. Numerous vectors are known in the art including, but not limited to, linear polynucleotides, polynucleotides associated with ionic or amphiphilic compounds, plasmids, and viruses. Thus, the term "vector" includes an autonomously replicating plasmid. The term should also be construed to include non-plasmid and non-viral compounds which facilitate transfer of nucleic acid into cells, such as, for example, polylysine compounds, liposomes, and the like. Examples of viral vectors include, but are not limited to, adenoviral vectors, adeno-associated virus vectors, retroviral vectors, and the like. Said vector may be preferentially a retroviral vector such as a lentiviral vector.

The terms "engineered cell" and "genetically modified cell" as used herein can be used interchangeably. The terms mean containing and/or expressing a foreign gene or nucleic acid sequence which in turn modifies the genotype or phenotype of the cell or its progeny. Especially, the terms refer to the fact that cells, preferentially T cells or NK cells can be manipulated by recombinant methods well known in the art to express stably or transiently peptides or proteins which are not expressed in these cells in the natural state.

The terms "nucleic acid", "nucleic acid sequence" or "polynucleotide" as used interchangeably herein refer to polymers of nucleotides. Polynucleotides, which can be hydrolyzed into monomeric "nucleotides." The monomeric nucleotides can be hydrolyzed into nucleosides. As used herein, the term "polynucleotides" encompasses, but is not limited to, all nucleic acid sequences which are obtained by any means available in the art, including, without limitation, recombinant means, i.e., the cloning of nucleic acid sequences from a recombinant library or a cell genome, using ordinary cloning technology and PCR, and the like, and by synthetic means. The T cell receptor (TCR) is a protein complex found on the surface of T cells, or T lymphocytes, that is responsible for recognizing fragments of antigen as peptides bound to major histocompatibility complex (MHC) molecules. However, TCRs can also be non-MHC-restricted, such as most γδ TCRs and TCRs employed by invariant natural killer T cells.

The TCR is composed of two different protein chains (that is, it is a heterodimer). In humans, in 95% of T cells the TCR consists of an alpha (α) chain and a beta (β) chain (encoded by TRA and TRB, respectively), whereas in 5% of T cells the TCR consists of gamma and delta (γ/δ) chains (encoded by TRG and TRD, respectively). This ratio changes during ontogeny and in diseased states (such as leukemia). Each locus can produce a variety of polypeptides with constant and variable regions.

The term "closed system" as used herein refers to any closed system which reduces the risk of cell culture contamination while performing culturing processes such as the introduction of new material, e.g. by transduction, and performing cell culturing steps such as proliferation, differentiation, activation, separation of cells, and/or electroporation if an in-line electroporation unit is connected. Such a system allows to operate under GMP or GMP-like conditions ("sterile") resulting in cell compositions which are clinically applicable. Herein exemplarily the CliniMACS Prodigy^{®} (Miltenyi Biotec B.V. & Co. KG, Germany) connected to the CliniMACS^{®} Electroporator (Miltenyi Biotec B.V. & Co. KG, Germany) is used as a closed system. The CliniMACS Prodigy^{®} is disclosed in WO2009/072003. But it is not intended to restrict the use of the method of the present invention to the CliniMACS Prodigy^{®}.

The process of the invention may be performed in a closed system, comprising a centrifugation chamber comprising a base plate and cover plate connected by a cylinder, pumps, valves, a magnetic cell separation column and a tubing set. The blood samples or other sources comprising NK cells may be transferred to and from the tubing set by sterile docking or sterile welding. A suitable system is disclosed in WO2009/072003.

The closed system may comprise a plurality of tubing sets (TS) where cells are transferred between TS by sterile docking or sterile welding.

Different modules of the process may be performed in different functionally closed TS with transfer of the product (cells) of one module generated in the one tubing set to another tubing set by sterile means. For example, NK cells can be magnetically enriched in a first tubing set (TS) TS 100 by Miltenyi Biotec and the positive fraction containing enriched NK cells is welded off the TS100 and welded onto a second tubing set TS730 by Miltenyi Biotec for further activation, modification, cultivation and washing.

The terms "automated method" or "automated process" as used herein refer to any process being automated through the use of devices and/or computers and computer software. Methods (processes) that have been automated require less human intervention and less human time. In some instances the method of the present invention is automated if at least one step of the present method is performed without any human support or intervention. Preferentially the method of the present invention is automated if all steps of the method as disclosed herein are performed without human support or intervention other than connecting fresh reagents to the system. Preferentially the automated process is implemented on a closed system such as CliniMACS Prodigy^{®} as disclosed herein.

The closed system may comprise a) a sample processing unit comprising an input port and an output port coupled to a rotating container (or centrifugation chamber) having at least one sample chamber, wherein the sample processing unit is configured to provide a first processing step to a sample or to rotate the container so as to apply a centrifugal force to a sample deposited in the chamber and separate at least a first component and a second component of the deposited sample; and b) a sample separation unit coupled to the output port of the sample processing unit, the sample separation unit comprising a separation column holder, a pump, and a plurality of valves configured to at least partially control fluid flow through a fluid circuitry and a separation column positioned in the holder, wherein the separation column is configured to separate labeled and unlabeled components of sample flown through the column.

Said rotating container may also be used as a temperature controlled cell incubation and cultivation chamber (CentriCult Unit = CCU). This chamber may be flooded with defined gas mixes, provided by an attached gas mix unit (e.g. use of pressurized air/ N2 / CO2 or N2/CO2/O2).

For enrichment, isolation or selection in principle any sorting technology can be used. This includes for example affinity chromatography or any other antibody-dependent separation technique known in the art. Any ligand-dependent separation technique known in the art may be used in conjunction with both positive and negative separation techniques that rely on the physical properties of the cells. An especially potent sorting technology is magnetic cell sorting. Methods to separate cells magnetically are commercially available e.g. from Invitrogen, Stem cell Technologies, in Cellpro, Seattle or Advanced Magnetics, Boston. For example, monoclonal antibodies can be directly coupled to magnetic polystyrene particles like Dynal M 450 or similar magnetic particles and used e.g. for cell separation. The Dynabeads technology is not column based, instead these magnetic beads with attached cells enjoy liquid phase kinetics in a sample tube, and the cells are isolated by placing the tube on a magnetic rack. However, in a preferred embodiment for enriching NK cells from a sample comprising NK cells according the present invention monoclonal antibodies or antigen binding fragments thereof are used in conjunction with colloidal superparamagnetic microparticles having an organic coating by e.g. polysaccharides (Magnetic-activated cell sorting (MACS) technology (Miltenyi Biotec B.V. & Co. KG, Germany)). These particles (nanobeads or MicroBeads) can be either directly conjugated to monoclonal antibodies or used in combination with anti-immunoglobulin, avidin or anti-hapten-specific MicroBeads.

The MACS technology allows cells to be separated by incubating them with magnetic nanoparticles coated with antibodies directed against a particular surface antigen. This causes the cells expressing this antigen to attach to the magnetic nanoparticles. Afterwards the cell solution is transferred on a column placed in a strong magnetic field. In this step, the cells attach to the nanoparticles (expressing the antigen) and stay on the column, while other cells (not expressing the antigen) flow through. With this method, the cells can be separated positively or negatively with respect to the particular antigen(s)/marker(s).

In case of a positive selection the cells expressing the antigen(s) of interest, which attached to the magnetic column, are washed out to a separate vessel, after removing the column from the magnetic field.

In case of a negative selection the antibody used is directed against surface antigen(s) which are known to be present on cells that are not of interest. After application of the cells/magnetic nanoparticles solution onto the column the cells expressing these antigens bind to the column and the fraction that goes through is collected, as it contains the cells of interest. As these cells are non-labelled by an antibody coupled to nanoparticles, they are "untouched".

The procedure can be performed using direct magnetic labelling or indirect magnetic labelling. For direct labelling the specific antibody is directly coupled to the magnetic particle. Indirect labelling is a convenient alternative when direct magnetic labelling is not possible or not desired. A primary antibody, a specific monoclonal or polyclonal antibody, a combination of primary antibodies, directed against any cell surface marker can be used for this labelling strategy. The primary antibody can either be unconjugated, biotinylated, or fluorophore-conjugated. The magnetic labelling is then achieved with anti-immunoglobulin MicroBeads, anti-biotin MicroBeads, or anti-fluorophore MicroBeads.

The term "cells that are CD3+" or "cells are CD3 positive" or "CD3 positive cells" may be used interchangeable and means that these cells are cells that express the antigen (marker) CD3 on their cell surface. This allows the select/sort cells that express this marker from cells that do not express this marker. It is understood that in the same way this applies to other markers such as CD14, CD19 or CD56.

The term "natural killer cells (NK cells)" are defined as large granular lymphocytes (LGL) and constitute the third kind of cells differentiated from the common lymphoid progenitor-generating B and T lymphocytes. NK cells are known to differentiate and mature in the bone marrow, lymph nodes, spleen, tonsils, and thymus, where they then enter the circulation. NK cells differ from natural killer T cells (NKTs) phenotypically, by origin and by respective effector functions; often, NKT cell activity promotes NK cell activity by secreting IFNγ. In contrast to NKT cells, NK cells do not express T-cell antigen receptors (TCR) or pan T marker CD3 or surface immunoglobulins (Ig) B cell receptors, but they usually express the surface markers CD16 (FcyRIII) and CD56 in humans, NK1.1 or NK1.2 in C57BL/6 mice. A subset of human NK cells also express CD8. Continuously growing NK cell lines can be established from cancer patients and common NK cell lines are for instance NK-92, NKL and YTS.

The term "isolated" means altered or removed from the natural state. For example an isolated population of cells means an enrichment of such cells and separation from other cells which are normally associated in their naturally occurring state with said isolated cells. An isolated population of cells means a population of substantially purified cells which are a homogenous population of cells.

As used herein the term "culturing" includes providing the chemical and physical conditions (e.g., temperature, gas) which are required for NK cell maintenance, and growth factors. Often culturing the NK cells includes providing the NK cells with conditions for expansion (proliferation). Examples of chemical conditions which may support NK cell expansion include but are not limited to buffers, serum, nutrients, vitamins, antibiotics, cytokines and other growth factors which are regularly provided in (or may be given manually to) the cell culture medium suited for NK cell expansion. In one embodiment, the NK cell culture medium includes TexMACS Research Medium (Miltenyi Biotec GmbH) supplemented with 5% human serum type AB (Life Technologies) and 500 U/mL of IL-2 (Proleukin S, Novartis). In another embodiment the NK cell culture medium includes Stem Cell Growth Medium SCGM (Cell Genix) supplemented with 5% human serum type AB (Life Technologies) and 500 U/mL of IL-2 (Proleukin S, Novartis). Other media suitable for use to expand NK cells are well known in the art. In another embodiment, the NK cell culture medium includes NK MACS Medium (Miltenyi Biotec, Germany) supplemented with 5% heat inactivated human serum type AB (Access Cell Culture, Vista, CA, USA), and together with cytokines, such as 500 U/mL of human IL-2 (Miltenyi Biotec, Germany), 140 U/mL of human IL-15 (Miltenyi Biotec, Germany). In some embodiments, feeder cells or artificial antigen presenting cells (APCs) such as K562-based feeder cells, lymphoblastoid cell lines, PMBCs, and/or cell-free derivatives such as cell particles or exosomes may be used to expand the composition enriched for NK cells. In some embodiments, the composition enriched for NK cells may be firstly primed with a cytokine from human IL-1 family, such as 2000 U/mL of IL-1beta and/or 50 ng/mL of IL-18 (Miltenyi Biotec, Germany) for 12 to 48 hours before expansion. In some embodiments, the composition enriched for NK cells may be firstly primed with 10 ng/ml of human IL-12, 50 ng/mL of human IL-18, and 50 ng/ml of human IL-15 (Miltenyi Biotec, Germany) for 12 to 18 hours before expansion. In some embodiments, the composition enriched for NK cells may be firstly stimulated with stimulating reagents with stimulating reagents, such as NK cell activation/expansion kit from Miltenyi Biotec, ImmunoCult^{™} NK Cell Expansion Kit from StemCell Technologies, CellXVivo Human NK Cell Expansion Kit from R&D Systems, before expansion.As used herein, the term "expansion" or "proliferation" refers to cell growth and multiplication of cell numbers. Expansion or proliferation, as used herein relate to increased numbers of NK cells occurring during the cultivation process.

The terms "culturing process" or "cultivation" as used herein refer to the culturing and expansion of cell such as NK cells. The culturing process may last as long as desired by the operator and can be performed as long as the cell culture medium has conditions which allow the cells to survive and/or grow and/or proliferate.

The cancer to be treated as disclosed herein , may be a solid cancer or may be a lymphoma or a hematological malignancy.

Said solid cancer (tumor) may be adrenal cancer, anal cancer, bile duct cancer, bladder cancer, bone cancer, brain/CNS tumors in children or adults, breast cancer, cervical cancer, colon/rectum cancer, endometrial cancer, esophagus cancer, ewing family of tumors, eye cancer, gallbladder cancer, gastrointestinal carcinoid tumors, gastrointestinal stromal tumor (GIST), gestation trophoblastic disease, hodgkin disease, kaposi sarcoma, kidney cancer, laryngeal and hypopharyngeal cancer, leukemia, acute lymphocytic leukemia, acute myeloid leukemia, chronic lymphocytic leukemia, chronic myeloid leukemia, chronic myelomonocytic leukemia, liver cancer, lung cancer, non-small cell lung cancer, small cell lung cancer, lung carcinoid tumor, lymphoma, malignant mesothelioma, multiple myeloma, myelodysplastic syndrome, nasal cavity and paranasal sinum cancer, nasopharyngeal cancer, neuroblastoma, non-hodgkin lymphoma, oral cavity or oropharyngeal cancer, osteosarcoma, ovarian cancer, pancreatic cancer, penile cancer, prostate cancer, rhabdomyosarcoma, , skin cancer, melanoma, merkel cell skin cancer, small intestine cancer, stomach cancer, testicular cancer, thyroid cancer, uterine sarcoma, vaginal cancer, vulvar cancer, or nephroblastoma.

Autoimmune diseases are a condition arising from autoimmunity or disbalance in the immune homeostasis resulting in pathologies that can affect multiple different organ systems. Examples include Behcet's disease, Juvenile idiopathic arthritis, Type 1 diabetes, Rheumatoid arthritis, Wegener Granulomatosis, Systemic lupus erythematosus, Systemic sclerosis, Crohn's disease, Graves' disease, Hashimoto thyroiditis, Goodpasture syndrome, Primary biliary cholangitis, Myasthenia gravis, Dermato polymyositis, Vasculitis, Mixed connective tissue disease, Scleroderma, Multiple sclerosis, Psoriasis, Ulcerative colitis and Uvetis.

Infection (infectious disease) is the invasion of an organism's body tissues by disease-causing agents, their multiplication, and the reaction of host tissues to the infectious agents and the toxins they produce. Infections are caused by infectious agents (pathogens) including: viruses, bacteria, fungi and parasites. Said infection may be an acute or a chronic infection.

Platelets (or thrombocytes) are a blood component whose function (along with the coagulation factors) is to react to bleeding from blood vessel injury by clumping, thereby initiating a blood clot. Resting platelets are small discoid shapes around 1.5-3 µm in size. Platelets have no cell nucleus; they are small fragments of cytoplasm derived from the megakaryocytes of the bone marrow or lung, which then enter the circulation. Platelets are found only in mammals.

### Embodiments

In one embodiment of the invention, the method comprises an in-vitro method for the generation of a composition of enriched NK cells using an obtained sample comprising leukocytes and platelets from a subject, wherein the leukocytes comprise at least NK cells and T cells, wherein the sample is a PBMC or a buffy coat or a leukapheresis or a cord blood from a subject, the method comprising
a) Depleting said platelets from said sample by washing by centrifugation, and
b) Depleting said T cells from the sample of a), wherein said depletion of T cells is performed by magnetic cell separation, and wherein said antigen binding domain specific for the antigen CD3 is directly coupled to magnetic beads.

Said washing by centrifugation may be performed by using a minimum g force between 25 x g and 200 x g, allowing to separate platelets from lymphocytes.

The composition enriched for NK cells may then be expanded with NK cell culture medium includes such as NK MACS Medium (Miltenyi Biotec, Germany) supplemented with 5% heat inactivated human serum type AB (Access Cell Culture, Vista, CA, USA), together with cytokines, such as 500 U/mL of human IL-2 (Miltenyi Biotec, Germany), 140 U/mL of human IL-15 (Miltenyi Biotec, Germany), to a composition comprising a therapeutically effective amount of NK cells. The composition comprising the expanded NK cells may be administered to a subject in need thereof, e.g. for use in treatment of acute myeloid leukemia (AML) or myelodysplastic syndrome (MDS). In some embodiments, feeder cells or artificial antigen presenting cells (APCs) such as K562-based feeder cells, lymphoblastoid cell lines, PMBCs, and/or cell-free derivatives such as cell particles or exosomes may be used to expand the composition enriched for NK cells. In some embodiments, the composition enriched for NK cells may be firstly primed with cytokines from human IL-1 family, such as 2000 U/mL of IL-1beta and/or 50 ng/mL of IL-18 (Miltenyi Biotec, Germany) before expansion. In some embodiments, the composition enriched for NK cells may be firstly primed with 10 ng/ml of human IL-12, 50 ng/mL of human IL-18, and 50 ng/ml of human IL-15 (Miltenyi Biotec, Germany) for 12 to 18 hours before expansion. In some embodiments, the composition enriched for NK cells may be firstly stimulated with stimulating reagents with stimulating reagents, such as NK cell activation/expansion kit from Miltenyi Biotec, ImmunoCult^{™} NK Cell Expansion Kit from StemCell Technologies, CellXVivo Human NK Cell Expansion Kit from R&D Systems, before expansion.

In one embodiment of the invention, the method comprises an in-vitro method for the generation of a composition of enriched NK cells using an obtained sample comprising leukocytes and platelets from a subject, wherein the leukocytes comprise at least NK cells and T cells, wherein the sample is a PBMC or a buffy coat or a leukapheresis or a cord blood from a subject, the method comprising
a) Depleting platelets from said sample, and
b) Depleting T cells from the sample of a) by depletion cells that are CD3+, and wherein the method additionally comprises:
   c1) further depleting cells that are CD19+ and/or CD14+, wherein the step c1) may be performed simultaneously with step b) or after step b), or
   c2) enriching cells after step b) that are CD56+.

Said washing by centrifugation may be performed by using a minimum g force between 25 x g and 200 x g, allowing to separate platelets from lymphocytes.

The composition enriched for NK cells may then be expanded with NK cell culture medium includes such as NK MACS Medium (Miltenyi Biotec, Germany) supplemented with 5% heat inactivated human serum type AB (Access Cell Culture, Vista, CA, USA), together with cytokines, such as and 500 U/mL of human IL-2 (Miltenyi Biotec, Germany), 140 U/mL of human IL-15 (Miltenyi Biotec, Germany), to a composition comprising a therapeutically effective amount of NK cells. The composition comprising the expanded NK cells may be administered to a subject in need thereof. In some embodiments, feeder cells or artificial antigen presenting cells (APCs) such as K562-based feeder cells, lymphoblastoid cell lines, PMBCs, and/or cell-free derivatives such as cell particles or exosomes may be used to expand the composition enriched for NK cells. In some embodiments, the composition enriched for NK cells may be firstly primed with a cytokines from human IL-1 family, such as 2000 U/mL of IL-1beta and/or 50 ng/mL of IL-18 (Miltenyi Biotec, Germany) for 12 to 48 hours before expansion. In some embodiments, the composition enriched for NK cells may be firstly primed with 10 ng/ml of human IL-12, 50 ng/mL of human IL-18, and 50 ng/ml of human IL-15 (Miltenyi Biotec, Germany) for 12 to 18 hours before expansion. In some embodiments, the composition enriched for NK cells may be firstly stimulated with stimulating reagents with stimulating reagents, such as NK cell activation/expansion kit from Miltenyi Biotec, ImmunoCult^{™} NK Cell Expansion Kit from StemCell Technologies, CellXVivo Human NK Cell Expansion Kit from R&D Systems, before expansion.

In one embodiment f the invention, the method comprises an in-vitro method for the generation of a composition of enriched NK cells using an obtained sample comprising leukocytes and platelets from a subject, wherein the leukocytes comprise at least NK cells and T cells, wherein

the sample is a PBMC or a buffy coat or a leukapheresis or a cord blood from a subject, the method comprising
a) Depleting said platelets from said sample by washing by centrifugation, and
b) Depleting said T cells from the sample of a), wherein said depletion of T cells is performed by magnetic cell separation, and wherein said antigen binding domain specific for the antigen CD3 is directly coupled to magnetic beads.

Said washing by centrifugation may be performed by using a minimum g force between 25 x g and 200 x g, allowing to separate platelets from lymphocytes.

Said composition enriched for NK cells may be administered directly, i.e. without further modification and expansion of the NK cells to a subject in need thereof. In some embodiments, the composition enriched for NK cells may be firstly primed with cytokines, such as IL-2, IL-15, IL-21, IL-12, IL-7, cytokines from human IL-1 family, and/or human cytokine derivatives, such as IL-15/IL-15R complex, prior to administering to a subject in need thereof. In some embodiments, the composition enriched for NK cells may be firstly primed with 10 ng/ml of human IL-12, 50 ng/mL of human IL-18, and 50 ng/ml of human IL-15 (Miltenyi Biotec, Germany) for 12 to 18 hours prior to administering to a subject in need thereof.

In one embodiment of the invention, , the method comprises an in-vitro method for the generation of a composition of enriched NK cells using an obtained sample comprising leukocytes and platelets from a subject, wherein the leukocytes comprise at least NK cells and T cells, wherein the sample is a PBMC or a buffy coat or a leukapheresis or a cord blood from a subject, the method comprising
a) Depleting platelets from said sample, and
b) Depleting T cells from the sample of a) by depletion cells that are CD3+, and wherein the method additionally comprises:
   c1) further depleting cells that are CD19+ and/or CD14+, wherein the step c1) may be performed simultaneously with step b) or after step b), or
   c2) enriching cells after step b) that are CD56+.

Said washing by centrifugation may be performed by using a minimum g force between 25 x g and 200 x g, allowing to separate platelets from lymphocytes.

Said composition enriched for NK cells may be administered directly, i.e. without further modification and expansion of the NK cells to a subject in need thereof. In some embodiments, the composition enriched for NK cells may be firstly primed with cytokines, such as IL-2, IL-15, IL-21, IL-12, IL-7, cytokines from human IL-1 family, and/or human cytokine derivatives, such as IL-15/IL-15R complex, prior to administering to a subject in need thereof. In some embodiments, the composition enriched for NK cells may be firstly primed with 10 ng/ml of human IL-12, 50 ng/mL of human IL-18, and 50 ng/ml of human IL-15 (Miltenyi Biotec, Germany) for 12 to 18 hours prior to administering to a subject in need thereof.

In one embodiment of the invention, the method comprises an automated method in a closed system such as the CliniMACS Prodigy^{®} (Miltenyi Biotec, Germany) for preparing a composition for subsequent processing comprising enriched NK cells using an obtained sample comprising leukocytes and platelets from a subject, wherein the leukocytes comprise at least NK cells and T cells, wherein the sample is a PBMC from a subject, the method comprising
a) Depleting said platelets from said sample by washing by centrifugation, and
b) Depleting said T cells from the sample of a), wherein said depletion of T cells is performed by magnetic cell separation, and wherein said antigen binding domain specific for the antigen CD3 is directly coupled to magnetic beads.

Said washing by centrifugation may be performed by using a minimum g force between 25 x g and 200 x g, allowing to separate platelets from lymphocytes.

The enriched NK cells may then be genetically modified in vitro by transduction with a lentiviral vector. Said lentiviral vector may comprise e.g. a nucleic acid encoding a chimeric antigen receptor (CAR). Said CAR may comprise an antigen binding domain specific for a tumor associated antigen (TAA). The modified NK cells expressing said CAR may be expanded in that closed system to a therapeutically effective amount. The expanded NK cells may be administered to a subject suffering from a cancer that is associated with aid TAA.

In one embodiment of the invention, the method comprises an automated method in a closed system such as the CliniMACS Prodigy^{®} (Miltenyi Biotec, Germany) for preparing a composition for subsequent processing comprising enriched NK cells using an obtained sample comprising leukocytes and platelets from a subject, wherein the leukocytes comprise at least NK cells and T cells, wherein the sample is a PBMC from a subject, the method comprising
a) Depleting platelets from said sample, and
b) Depleting T cells from the sample of a) by depletion cells that are CD3+, and wherein the method additionally comprises:
   c1) further depleting cells that are CD19+ and/or CD14+, wherein the step c1) may be performed simultaneously with step b) or after step b), or
   c2) enriching cells after step b) that are CD56+.

Said washing by centrifugation may be performed by using a minimum g force between 25 x g and 200 x g, allowing to separate platelets from lymphocytes.

The enriched NK cells may then be genetically modified in vitro by transduction with a lentiviral vector. Said lentiviral vector may comprise e.g. a nucleic acid encoding a chimeric antigen receptor (CAR). Said CAR may comprise an antigen binding domain specific for a tumor associated antigen (TAA). The modified NK cells expressing said CAR may be expanded in that closed system to a therapeutically effective amount. The expanded NK cells may be administered to a subject suffering from a cancer that is associated with aid TAA.

In one embodiment of the invention, the method comprises an in-vitro method for the generation of a composition of enriched NK cells using an obtained sample comprising leukocytes and platelets from a subject, wherein the leukocytes comprise at least NK cells and T cells, wherein the sample is a PBMC or a buffy coat or a leukapheresis or a cord blood from a subject, the method comprising
a) Depleting said platelets from said sample by washing by centrifugation, and
b) Depleting said T cells from the sample of a), wherein said depletion of T cells is performed by magnetic cell separation, and wherein said antigen binding domain specific for the antigen CD3 is directly coupled to magnetic beads.

Said washing by centrifugation may be performed by using a minimum g force between 25 x g and 200 x g, allowing to separate platelets from lymphocytes.

The enriched NK cells may then be genetically modified in vitro by transduction with a lentiviral vector. Said lentiviral vector may comprise e.g. a nucleic acid encoding a chimeric antigen receptor (CAR). Said CAR may comprise an antigen binding domain specific for a tumor associated antigen (TAA). The modified NK cells expressing said CAR may be expanded to a therapeutically effective amount. The expanded NK cells may be administered to a subject suffering from a cancer that is associated with aid TAA.

In one embodiment of the invention, the method comprises an in-vitro method for the generation of a composition of enriched NK cells using an obtained sample comprising leukocytes and platelets from a subject, wherein the leukocytes comprise at least NK cells and T cells, wherein the sample is a PBMC or a buffy coat or a leukapheresis or a cord blood from a subject, the method comprising
a) Depleting platelets from said sample, and
b) Depleting T cells from the sample of a) by depletion cells that are CD3+, and wherein the method additionally comprises:
   c1) further depleting cells that are CD19+ and/or CD14+, wherein the step c1) may be performed simultaneously with step b) or after step b), or
   c2) enriching cells after step b) that are CD56+.

Said washing by centrifugation may be performed by using a minimum g force between 25 x g and 200 x g, allowing to separate platelets from lymphocytes.

The enriched NK cells may then be genetically modified in vitro by transduction with a lentiviral vector. Said lentiviral vector may comprise e.g. a nucleic acid encoding a chimeric antigen receptor (CAR). Said CAR may comprise an antigen binding domain specific for a tumor associated antigen (TAA). The modified NK cells expressing said CAR may be expanded to a therapeutically effective amount. The expanded NK cells may be administered to a subject suffering from a cancer that is associated with aid TAA.

### Examples

The following examples are intended for a more detailed explanation of the invention but without restricting the invention to these examples.

### Example 1: The CD3+ cell log depletion is negatively correlated with the ratio of platelets to T cells in the sample.

Leukaphereses or buffy coats acquired from healthy donors were washed with CliniMACS^{®} PBS/EDTA Buffer (Miltenyi Biotec) in CliniMACS Prodigy^{®} Instrument (Miltenyi Biotec), resulting in the ratio of platelets to T cells in the range from 20 to 424, CD3+ T cells were then depleted with CliniMACS^{®} CD3 Reagent (Miltenyi Biotec) by using the CliniMACS Prodigy^{®} LP-3-56 System (Miltenyi Biotec).

At each step, the number of platelets were determined by using XP-300 hematology analyser (Sysmex) and the numbers of CD3+ T cells were determined by flow cytometric analysis in MACSQuant^{®} Analyzer 10 flow cytometer (Miltenyi Biotec). The log-depletion of CD3+ T cells was calculated as log 10 (total CD3+ T cell number after depletion/total CD3+ T cell number before depletion). Surprisingly, there is a significant negative correlation between the ratio of platelets to T cells in the samples after sample preparation and the log-depletion degree of CD3+ T cells (Figure 1).

### Example 2: Minimum g-force is a critical Parameter in determining platelet removal efficiency.

In this example, washing by centrifugation was used to remove platelets in blood samples. A critical parameter for separation efficiency in centrifugation is the relative centrifugal force (RCF) or g-force, which is expressed in units of gravity (times gravity or x g). G-force exerted on samples in a centrifuge is a function of the rotation speed of the centrifuge and the distance from the axis of rotation of the centrifuge (radius).

The minimum, average, or maximum g-force can be calculated from the minimum, average, or maximum radius, which are determined by the distance between the axis of rotation of a centrifuge and the top, middle, or bottom of a sample,

The minimum radius and the maximum radius are sometimes referred to as the inner radius and outer radius, respectively. The radius measurement is shown for a centrifuge with a rotor (Figure 2A) or a centrifugation chamber, such as those used on the CliniMACS Prodigy^{®} platform (Figure 2B).

Human peripheral blood mononuclear cells (PBMCs) were divided into two equal volumes of blood and each half of the blood sample was loaded separately into a different centrifugation chamber of CliniMACS Prodigy^{®} Tubing Set 320 (TS 320) (Miltenyi Biotec). To remove platelets in the blood samples, the same maximum g force of 322 x g was applied to both samples in the centrifugation chamber.

However, the minimum g-force for each sample was set to 258.4 x g and 125.1 x g by adjusting the volumes of the samples with CliniMACS^{®} PBS/EDTA Buffer, respectively. Intriguingly, the sample that was centrifuged with the minimum g-force of 125.1 x g showed more efficient platelet removal of more than 30%, compared with the sample that was centrifuged with the minimum g-force of 258.4 x g, in which only less than 5% platelets were removed (Figure 3). This example demonstrates the importance of minimum g-force for efficient platelet removal in blood samples by centrifugation washing.

### Example 3: Optimization of the centrifugation washing method to improve the platelet removal efficiency.

In this example, different minimum g-forces were applied to blood
samples to optimize the platelet removal. The efficiency of platelet removal for each washing round was also determined.

In one experiment, a buffy coat from a healthy donor was divided into two equal volumes of blood, and each half of the blood sample was loaded separately into a different centrifugation chamber of CliniMACS Prodigy^{®} Tubing Set 620 (TS 620) (Miltenyi Biotec). The minimum g-force for each sample was set to 59.2 x g and 164.3 x g, respectively. The sample that was centrifuged with the minimum g-force of 164.3 x g showed a platelet removal efficiency of 32%. Intriguingly, a higher platelet removal efficiency of 62.3% was observed for the sample that was centrifuged with the lower minimum g-force of 59.2 x g (Figure 4A).

In another experiment, a comparison between the minimum g-force of 59.2 x g and 80.3 x g was performed for buffy coats from two donors. Similarly high mean platelet removal efficiencies of 68.8% and 59.4% were obtained for both minimum g-force settings, respectively (Figure 4B). Three washing rounds were applied to one buffer coat sample centrifuged with the minimum g-force of 80.3 x g. The platelet removal efficiency was calculated after each wash based on the amount of platelets in the starting blood material, resulting in 65% and 23% of platelets being depleted from the blood sample in the first and second wash, respectively. However, only 3.4% of platelet removal was achieved in the third wash (Figure 4C).

In another experiment, a comparison between the minimum g-force of 223.0 x g and 80.3 x g was performed for leukaphereses from two donors. The samples that were centrifuged with the minimum g-force of 223.0 x g showed a mean platelet removal efficiency of 25.5%. A higher mean platelet removal efficiency of 51.7% was observed for the samples that were centrifuged with the lower minimum g-force of 80.3 x g (Figure 4D).

This example further demonstrates the importance of minimum g-force applied to blood samples for efficient platelet removal by centrifugation washing method. In addition, the majority of platelets can be efficiently removed from blood samples with the first and second wash.

### Example 4: Efficient platelet removal can be achieved by magnetic cell separation technology.

Platelets can also be removed with magnetic cell Separation technology. In this Example, CD61 MicroBeads, human (Miltenyi Biotec) was used to deplete platelets from a leukapheresis sample according to the manufacturer's Instruction. A platelet removal efficiency of 98% was achieved by CD61 MicroBeads (Figure 5).

## Claims

1. A method for generating a composition comprising NK cells using an obtained sample comprising leukocytes and platelets from a subject, wherein the leukocytes comprise at least NK cells and T cells, the method comprising
a) Depleting platelets from said sample, and
b) Depleting T cells from the sample of a).

2. The method of claim 1, wherein the ratio of platelets to T cells in the sample that is depleted of platelets is less than 150.

3. The method of claim 1 to 2, wherein said depleting of platelets from said sample is performed by
i) Washing by centrifugation, or
ii) Affinity chromatography using CD61 as a selection marker

4. The method of claim 3, wherein said washing by centrifugation is performed in such a way that in said sample comprising leukocytes and platelets the speed of rotation is lower than that used to pellet the platelets but pellets the lymphocytes.

5. The method of claim 3, wherein the depletion of T cells is performed by using an antigen binding domain specific for the antigen CD3 or specific for the alpha/beta T cell receptor (TCR).

6. The method of claim 5, wherein said depletion of T cells is performed by magnetic cell separation, and wherein said antigen binding domain specific for the antigen CD3 or for the alpha/beta TCR is directly or indirectly coupled to magnetic beads.

7. The method of claim 3, wherein said affinity chromatography is performed by using an antigen binding domain specific for the antigen CD61.

8. The method of claim 7, wherein said affinity chromatography is performed by magnetic cell separation, and wherein said antigen binding domain specific for the antigen CD61 is directly or indirectly coupled to magnetic beads.

9. The method of any one of claims 1 to 8, wherein the method is performed in a closed system.

10. The method of claim 9, wherein said method is an automated method.

11. The method of any one of claims 1 to 10, wherein the method comprises additionally
c) Genetically modifying of the NK cells of step b).

12. The method of claim 11, wherein said genetically modifying of the NK cells is the transduction of said NK cells with a lentiviral vector.

13. The method of claim 12, wherein aid lentiviral vector comprises a nucleic acid encoding a chimeric antigen receptor (CAR).

14. A composition comprising NK cells, wherein said composition is obtained by the methods of any one of claims 1 to 13.

15. The composition of claim 14, wherein said composition is a pharmaceutical composition.
